# EUROPÄISCHE PATENTSCHRIFT

(11) **EP 2 269 718 B1**
(45) Veröffentlichungstag und Bekanntmachung des Hinweises auf die Patenterteilung: **31.07.2013**
(21) Anmeldenummer: 10005897.3
(22) Anmeldetag: 08.06.2010
(51) Int. Cl.: B01F 11/00, B01F 13/00, B01F 13/06, B01F 15/02, A61F 2/46

(54) **Vorrichtung zum Mischen und Austragen von Knochenzement, und Knochenzementsystem**
Device for mixing and applying bone cement, and bone cement system
Dispositif de mélange et d'extraction de ciment osseux, et système de ciment osseux

(30) Priorität: 29.06.2009 DE 102009031178
(43) Veröffentlichungstag der Anmeldung: 05.01.2011
(73) Patentinhaber: Heraeus Medical GmbH, 61273 Wehrheim (DE)
(72) Erfinder: Vogt, Sebastian, Dr., 99092 Erfurt (DE); Büchner, Hubert, Dr., 64354 Reinheim (DE)
(74) Vertreter: Kühn, Hans-Christian

(56) Entgegenhaltungen:
- EP-A2- 1 920 738
- WO-A1-00/35506
- US-A- 3 195 778
- US-A1- 2006 274 601
- US-B1- 6 488 651

## Beschreibung

Die Erfindung betrifft eine Vorrichtung zum Mischen und Austragen von Knochenzement gemäß den Merkmalen des Anspruchs 1, mit einem Mischzylinder, in weichem ein Mischkolben angeordnet ist, wobei der Mischkolben mittels einer an einem ersten Zylinderende gedichtet herausgeführten Betätigungsstange axial bewegbar ist. Weiterhin betrifft die Erfindung ein Knochenzementsystem mit den Merkmalen des Patentanspruchs 9.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). PMMA-Knochenzemente bestehen aus einer flüssigen Monomerkomponente und einer Pulverkomponente. Die Monomerkomponente enthalt im Allgemeinen das Monomer Methylmethacrylat und einen darin gelösten Aktivator (N,N-Dimethyl-p-toluidin). Die Pulverkomponente, auch als Knochenzementpulver bezeichnet, weist einen oder mehrere Polymeren, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Mothylmothacrylates. Mit fortschreitender Polymerisation des Methylmethacrylates erhöht sich die Viskositat des Zementteigs, bis dieser erstarrt.

Polymethylmethacrylat-Knochenzemente können in geeigneten Mischbechem mit Hilfe von Spateln durch Vermischen des Zementpulvers mit der Monomertlüssigkeit vermischt werden. Nachteilig ist an dieser Vorgehensweise, dass Lufteinschlüsse im gebildeten Zementteig vorhanden sein können, die später eine Destabilisierung des Knochenzements verursachen können. Aus diesem Grund wird das Vermischen von Knochenzementpulver mit der Monomerfossigkeit in Vakuummischsystemen bevorzugt, weil durch Mischen im Vakuum Lufteinschlüsse aus dem Zementteig weitgehend entfernt werden und damit eine optimale Zementqualität erreicht wird (Breusch SJ et al.: Der Stand der Zementiertechnik in Deutschland, Z. Orthop. 1999, 137, 101-07). Im Vakuum gemischte Knochenzemente haben eine deutlich verringerte Porosität und zeigen daher verbesserte mechanische Eigenschaften. Es wurden eine Vielzahl von Vakuum-Zementiersystemen offen gelegt, von denen exemplarisch folgende genannt sind: US 6,033,105 A, US 5,624,184 A, US 4,671,263 A, US 4,973,168 A, US 5,100,249 A, WO 99/67015 A1, EP 1 020 167 A2, US 5,586,821 A, EP 1 016 452 A2, DE 36 40 279 A1, WO 94/26403 A1, EP 0 692 229 A1, EP 1005 901 A2, US 5,344,232 A. Ebenfalls beschreibt die EP 1 920 738 A2 ein Vakuum-Zementiersystem, mittels dessen Knochenzemente erstellt werden können.

Weitere Zementiersysteme sind aus den Dokumenten US 2006/274601 A1, EP 1 920 738 A2, US 3,195,778 A sowie US 6,488,651 B1 bekannt, wobei US 2006/274601 eine Vorrichtung gemäβ den oberbegriff des Anspruchs 1 offenbart.
Eine Weiterentwicklung stellen Zementiersysteme dar, in denen sowohl das Zementpulver als auch die Monomerflüssigkeit bereits in separaten Kompartimenten der Mischsysteme verpackt sind und erst unmittelbar vor der Zementapplikation im Zementiersysteme miteinander vermischt werden (EP 0 692 229 A1). Ein wesentliches Problem dieser Systeme ist die Sterilisation des gesamten Systems einschließlich des Zementpulvers und der zuvor steril filtrierten Monomerflüssigkeit. Problematisch ist insbesondere die Durchführung der bei Knochenzementen häufig genutzten Sterilisation mit Ethylenoxid. Diese Sterilisationsmethode hat gegenüber der Sterilisation mit Gammastrahlen den Vorteil, dass die im Zementpulver enthaltenen Polymere nicht degradiert werden und die Zementeigenschaften durch die Ethylenoxid-Sterilisation unbeeinflusst bleiben. Problematisch ist bei der Sterilisation mit Ethylen oxid, da das gasförmige Agens zuerst in die Kartusche bzw. den Zementvorratsbehälter und damit in das Zementpulver eindringen muss und nach der Sterilisation wieder aus der Kartusche heraus diffundieren muss. Ein möglichst ungehinderter Gasaustausch zwischen dem Inneren der Kartusche bzw. des Vorratsbehälters und der Umgebung ist daher zwingend erforderlich. Im Widerspruch dazu muss das einsatzbereite Mischsystem so dicht verschlossen sein, dass ein Anmischen des Zements unter Vakuum erfolgen kann.

Bei auf dem Markt befindlichen Mischsystemen wird dieser Widerspruch dadurch gelöst, dass auf der Zementkartusche ein Deckel mit einer porösen Scheibe aufgeschraubt ist, der unmittelbar vor der Zementapplikation entfernt werden muss. Anstelle dieses Deckels wird ein vakuumdichter Kartuschenkopf aufgeschraubt, der eine Mischvorrichtung, einen Vakuumanschluss und eine Öffnung für das später aufzusetzende Austragsrohr enthält. Der medizinische Anwender muss daher das Zementiersystem unmittelbar vor der Zementanmischung öffnen und dann wieder verschließen. Dadurch können Keime etc. an das zuvor desinfizierte Knochenzementpulver gelangen.

Es ist die Aufgabe der vorliegenden Erfindung, eine Vorrichtung zum Mischen und Austragen von Knochenzement zu schaffen, bei der die genannten Nachteile vermieden werden, insbesondere eine Verunreinigung des desinfizierten Knochenzementpulvers verhindert wird.

Zur Lösung dieser Aufgabe wird eine Vorrichtung zum Mischen und Austragen von Knochenzement mit den Merkmalen des Anspruchs 1 vorgeschlagen. Weiterhin wird zur Lösung dieser Aufgabe ein Knochenzementsystem mit den Merkmalen des Anspruchs 9 vorgeschlagen. In den abhängigen Ansprüchen sind jeweils bevorzugte Weiterbildungen ausgeführt. Merkmale und Details, die in Zusammenhang mit der Vorrichtung offenbart werden, gelten dabei selbstverständlich auch für das erfindungsgemäße Knochenzementsystem und umgekehrt.

Die Erfindung betrifft eine Vorrichtung zum Mischen und Austragen von Knochenzement, mit einem Mischzylinder, in welchem ein Mischkolben angeordnet ist, wobei der Mischkolben mittels einer an einem ersten Zylinderende gedichtet herausgeführten Betätigungsstange axial bewegbar ist, und mit einem im Bereich des ersten Zylinderendes angeordneten und auf der Betätigungsstange axial bewegbaren Dichtungskolben, der den Mischzylinder gasdicht abschließt, wobei im Bereich des ersten Zylinderendes zwischen dem Mischkolben und dem Dichtungskolben ein Sterilisationskolben angeordnet ist, der getrennt von dem Dichtungskolben auf der Betätigungsstange axial bewegbar ist, und den Mischzylinder gasdurchlässig abschließt, wobei der Sterilisationskolben mit dem Dichtungskolben ein zweiteiliges Kolbensystem bildet, wobei in einer Mischstellung der Dichtungskolben den Mischzylinder gasdicht abschließt, um ein Durchspülen des Knochenzementpulvers mit einem Binder, insbesondere einem Monomer, zu ermöglichen.

Die Erfindung ist dadurch gekennzeichnet, dass der Sterilisationskolben ein gasdurchlässiges Gitter aufweist, das Partikel, die kleiner als 5 µm sind, durchlässt, und in einer Sterilisationsstellung der Sterilisationskolben den Mischzylinder gasdurchlässig abschließt, um ein Durchspülen eines Knochenzementpulvers mit einem Sterilisationsmittel zu ermöglichen.

Der Sterilisationskolben ist zwischen dem Mischkolben und dem Dichtungskolben auf der Betätigungsstange angeordnet. Durch diese Anordnung der zwei Teile des Kolbensystems ist sichergestellt, dass ein Sterilisationsmittel in den Mischzylinder einströmen kann, ohne von dem Dichtungskolben daran gehindert zu werden. Erst im Anschluss wird der Dichtungskolben auf den Sterilisationskolben aufgebracht und somit das zweiteilige Kolbensystem kombiniert.

Der Kern der hier beschriebenen Erfindung besteht darin, ein Ende des Mischzylinders mit einem zweiteiligen Kolbensystem zu verschließen. Die zwei Teile des Kolbensystems können unabhängig voneinander auf der Betätigungsstange axial verschoben werden. Der Sterilisationskolben verschließt den Mischzylinder gasdurchlässig. Somit kann ein Sterilisationsmittel in den Mischzylinder einströmen und das dort gelagerte Knochenzementpulver sterilisieren. Der Dichtungskolben des Kolbensystems ermöglicht dann einen gasdichten Abschluss des Mischzylinders, um diesen mit einem Vakuum zu verbinden und einen Binder für das Knochenzementpulver in den Mischkolben einzusaugen. Das zweiteilige Kolbensystem zeichnet sich dadurch aus, dass der Sterilisationskolben den Mischzylinder gasdurchlässig abschließt, um ein Durchspülen eines Knochenzementpulvers mit einem Sterilisationsmittels zu ermöglichen und der Dichtungskolben den Mischzylinder gasdicht abschließt, um ein Durchspülen des Knochenzementpulvers mit einem Binder, insbesondere einem Monomer zu ermöglichen.

Die Besonderheit besteht darin, dass einerseits eine möglichst große gaspermeable Fläche im Zementmischsystem vorhanden ist, damit Ethylenoxid zur Sterilisation einströmen kann und nach erfolgter Sterilisation wieder ausgasen kann und dass andererseits ein vakuumdichter Verschluss vorhanden ist, damit ein Vakuum-Anmischen des Knochenzements in der verschlossenen Kartusche möglich ist. Das Kolbensystem kann gleichzeitig als Kolben zum Austragen des gemischten Zements aus der Kartusche verwendet werden. Das zweiteilige Kolbensystem erfüllt somit insgesamt drei Vorteile: Zum ersten ist es möglich, durch eine Abdichtung des Mischzylinders durch den Sterilisationskolben das Knochenzementpulver mit einem gasförmigen Fluid zu desinfizieren. Im Anschluss wird der Dichtungskolben genutzt, um den Sterilisationskolben abzudichten. Dadurch ist ein Mischen des Knochenzementpulvers mit einem Binder, insbesondere einem Monomer, unter Vakuum möglich. Anschließend wird das aus Dichtungskolben und Sterilisationskolben gebildete Kolbensystem genutzt, um in den Mischzylinder eingeschoben zu werden und dadurch den Knochenzement aus diesem auszupressen.

Im Rahmen der Erfindung soll der Dichtungskolben den Mischzylinder gasdicht abschließen. Damit soll verdeutlicht werden, dass der Dichtungskolben den Mischzylinder gegenüber den beim Mischen auftretenden und dem Fachmann bekannten Drücken abschließt. Das in den Mischzylinder mittels einer Vakuumpumpe eingebrachte Vakuum soll nicht wesentlich durch etwaige Leckage des Dichtungskolbens gegenüber dem Mischzylinder geschwächt werden. Im Rahmen der Erfindung soll der Begriff "gasdurchlässig" verdeutlichen, dass ein gasförmiges Sterilisationsfluid durch den Sterilisationskolben in den Mischzylinder einströmen kann. Der Sterilisationskolben weist ein gasdurchlässiges Gitter auf, das Partikel, die kleiner als 5 µm sind, durchlässt. Größere Partikel sollten von dem gasdurchlässigen Sterilisationskolben nicht in das Innere des Mischzylinders gelassen werden.

Eine vorteilhafte Ausführungsvariante der erfindungsgemäßen Vorrichtung zeichnet sich dadurch aus, dass das zweiteilige Kolbensystem die Betätigungsstange umschließt. Dabei hat es sich als besonders vorteilhaft herausgestellt, wenn das Kolbensystem und/oder der Sterilisationskolben und/oder der Dichtungskolben zylinderartig ausgestaltet sind. Durch die Anordnung des Kolbensystems um die Betätigungsstange ist ein einfaches und reversibles Verschieben des Dichtungskolbens auf den Sterilisationskolben zu bzw. von diesem weg möglich. In einer Sterilisationsstellung ist der Sterilisationskolben in dem Mischzylinder angeordnet, während der Dichtungskolben beispielsweise außerhalb des Mischzylinders, aber auf der Betätigungsstange angeordnet ist. So ist sichergestellt, dass ein Sterilisationsmittel ungehindert in das Innere des Mischzylinders einströmen kann. In einer Mischsituation wird dann der Dichtungskolben auf den Sterilisationskolben aufgebracht, so dass das Kolbensystem zusammen angeordnet ist. Durch den Dichtungskolben wird verhindert, dass das im Inneren des Mischzylinders herrschende Vakuum geschwächt werden kann.

Um eine kraft- und/oder formschlüssige Verbindung zwischen dem Dichtungskolben und dem Sterilisationskolben herzustellen, hat es sich als bevorzugt herausgestellt, wenn der Dichtungskolben auf den Sterilisationskolben aufsteckbar ist. Beide Kolben des Kolbensystems können über entsprechende Clipverbindungen verfugen, so dass der Sterilisationskolben auf den Dichtungskolben aufsteckbar ist. Diese Clipverbindung kann reversibel lösbar ausgestaltet sein, um ein mehrfaches Aufbringen des Sterilisationskolbens zu ermöglichen.

Eine Besonderheit der erfindungsgemäßen Vorrichtung besteht darin, dass durch die Aufteilung des Kolbensystems in einen Dichtungskolben und einen Sterilisationskolben nicht nur das Knochenzementpulver problemlos und ohne die Gefahr einer Verunreinigung desinfiziert werden kann, sondern auch ein Austragen des fertigen Knochenzements mit dem zweiteiligen Kolbensystem möglich ist. So hat es sich als vorteilhaft erwiesen, wenn das Kolbensystem axial in den Mischzylinder eindrückbar ist, um einen aus dem Knochenzementpulver und dem Binder, insbesondere dem Monomer, gemischten Knochenzement, durch eine Austragsöffnung auszutragen. Die Austragsöffnung liegt an einem zweiten Zylinderende des Mischzylinders. Das zweite Zylinderende ist dem ersten Zylinderende entgegengesetzt. Beim Austragen wird das Kolbensystem aus Richtung des ersten Zylinderendes in Richtung des zweiten Zylinderendes gedrückt und presst dabei den fertig gemischten Knochenzement durch die Austragsöffnung heraus. In einer vorteilhaften Ausgestaltung weist die Austragsöffnung ein Anschlussmittel, insbesondere ein Anschlussgewinde, auf. Dieses Anschlussgewinde kann genutzt werden, urn den Mischzylinder in das noch zu beschreibende Knochenzementsystem einzuschrauben und/oder den Mischzylinder an ein Schlauchsystem anzuschließen, über das der fertige Knochenzement in den Knochen eingebracht werden kann. Für diese Tätigkeit kann eine Auspresspistole genutzt werden, in die der Mischzylinder eingespannt wird. Zur einfachen Benutzung der Auspresspistole kann die Betätigungsstange eine Sollbruchstelle aufweisen, so dass ein Abbrechen der Betätigungsstange an einer definierten Stelle möglich ist. Zum Austragen des fertig gemischten Knochenzements wird die Betätigungsstange in Richtung des Kolbensystems gezogen, bis der Mischkolben an dem Kolbensystem anliegt. Durch das dann erfolgende Abtrennen der Betätigungsstange kann das Kolbensystem mit dem davor anliegenden Mischkolben in den Mischzylinder eingepresst werden.

Um das Knochenzementpulver in das Innere des Mischzylinders einzufüllen, hat es sich als vorteilhaft erwiesen, wenn der Sterilisationskolben eine verschließbare, insbesondere reversibel verschließbare Öffnung aufweist. Mittels dieser Befüllöffnung ist ein leichtes Einbringen des Knochenzementpulvers in den Mischzylinder möglich, ohne dass das Kolbensystem aus dem Mischzylinder entnommen werden muss. Dadurch wird eine Reduktion des Aufwandes zum Befallen der erfindungsgemäßen Vorrichtung erreicht.

Weiterhin hat es sich als vorteilhaft herausgestellt, wenn der Sterilisationskolben und/oder der Dichtungskolben wenigstens ein Führungsmittel aufweist, welches zur Abstützung gegenüber der Betätigungsstange dient. Durch das Führungsmittel wird verhindert, dass einer der genannten Kolben des Kolbensystems sich in dem Mischzylinder verkantet. Diese Verkantung könnte insbesondere dann auftreten, wenn der Dichtungskolben entlang der Betätigungsstange gefahren wird, um auf dem Sterilisationskolben aufgebracht zu werden. Weiterhin ist es vorteilhaft, wenn das Kolbensystem und/oder der Sterilisationskolben und/oder der Dichtungskolben wenigstens ein Abdichtungsmittel aufweisen, um eine Abdichtung gegenüber dem Mischzylinder zu erzielen. Der Dichtungskolben soll eine gasdichte Abdichtung des Mischzylinders erzielen. Zusätzlich ist geplant, dass der Sterilisationskolben nur Partikel kleiner als 5 µm in den Mischzylinder einströmen lässt. Um diese Anforderung zu erfüllen, hat es sich als vorteilhaft erwiesen, wenn ein Abdichtungsmittel auf einer Außenfläche, die in Kontakt mit dem Mischzylinder steht, über ein Abdichtungsmittel verfügt. Dieses Abdichtungsmittel kann beispielsweise eine Gummidichtung sein, die verhindert, dass Umgebungsluft in das Innere des mit einem Vakuum versehenen Mischzylinders einströmt.

Eine weitere vorteilhafte Ausgestaltung der erfindungsgemäßen Vorrichtung zeichnet sich dadurch aus, dass das Kolbensystem und/oder der Sterilisationskolben und/oder der Dichtungskolben wenigstens einen Vakuumanschluss aufweist. Über diesen Vakuumanschluss kann die Vorrichtung mit einer Vakuumpumpe verbunden werden. Die Vakuumpumpe erzeugt dann jenen Unterdruck, der in dem Mischzylinder vorherrschen soll. Wie später noch ausführlich beschrieben wird, sorgt der Unterdruck für ein Einfließen des Binders, insbesondere des Monomers, in das Knochenzementpulver. Die Anordnung des Vakuumanschlusses in dem Kolbensystem hat sich aufgrund der leichten Erreichbarkeit als besonders vorteilhaft herausgestellt.

Die oben genannte Aufgabe wird ebenfalls gelöst durch ein Knochenzementsystem entsprechend Anspruch 9. Merkmale und Details, die dabei in Zusammenhang mit der Vorrichtung beschrieben wurden, gelten auch im Zusammenhang mit dem Knochenzementsystem und umgekehrt, denn das Knochenzementsystem beinhaltet die erfindungsgemäße Vorrichtung mit dem erfindungsgemäß zweiteilig ausgelegten Kolbensystem. Dabei zeichnet sich das Kolbensystem derart aus, dass der Sterilisationskolben den Mischzylinder gasdurchlässig abschließt, um ein Durchspülen eines Knochenzementpulvers mit einem Sterilisationsmittels zu ermöglichen und der Dichtungskolben den Mischzylinder gasdicht abschließt, um ein Durchspülen des Knochenzementpulvers mit einem Binder, insbesondere einem Monomer, zu ermöglichen.

Eine vorteilhafte Ausgestaltung des erfindungsgemäßen Knochenzementsystems zeichnet sich dadurch aus, dass das Basiselement ein Koppelmittel aufweist für eine kraft- und/oder formschlüssige Verbindung mit der Vorrichtung, insbesondere einer Austragsöffnung der Vorrichtung. Das Basiselement dient als Lager sowohl für die erfindungsgemäße Vorrichtung, als auch das Vorratselement für den Binder. Als eine Art Fundament des Knochenzementsystems können die erfindungsgemäße Vorrichtung und das Vorratselement an und/oder auf dem Basiselement angeordnet sein. Da die erfindungsgemäße Vorrichtung auch zum Austragen des Knochenzements genutzt werden soll, ist es vorteilhaft, wenn die Vorrichtung reversibel von dem Basiselement trennbar ist. Dies kann durch das erfindungsgemäße Koppelement erreicht werden. Vorteilhafterweise handelt es sich bei dem Koppelelement um ein Gewinde, auf welches die Austragsöffnung der Vorrichtung aufgeschraubt werden kann. So ist eine sichere Verbindung zwischen dem Basiselement und der Vorrichtung gewährleistet.

Weiterhin ist es vorteilhaft, wenn das Vorratselement einen Vorratsbehälter für den Binder, insbesondere den Monomer, lagert. Zur Herstellung des Knochenzements muss der Binder, insbesondere der Monomer, in das Knochenzementpulver eingebracht werden. Nach einer gewissen Zeitspanne härtet der Knochenzement dann aus. Offensichtlich kann der Knochenzement somit nicht austragsfertig in der Vorrichtung ausgeliefert werden. Es ist somit notwendig, dass das Knochenzementpulver und der Binder, insbesondere der Monomer, bis kurz vor dem Austragen des Knochenzements getrennt gelagert werden müssen. Somit bietet es sich an, wenn das Vorratselement einen Vorratsbehälter für den Binder, insbesondere den Monomer, aufweist. Als leicht zu desinfizieren haben sich insbesondere Glasbehälter herausgestellt, die als Vorratsbehälter für den Binder, insbesondere den Monomer, genutzt werden. Um einen Zufluss des Monomers zu steuern, kann das Vorratselement ein Ventilmittel aufweisen. Dieses Ventilmittel steuert und/oder löst den Zufluss des Monomers aus dem Vorratsbehälter in die erfindungsgemäße Vorrichtung aus.

Damit der Binder, insbesondere der Monomer, aus dem Vorratselement in die Vorrichtung strömen kann, weist das Basiselement ein Leitungsmittel auf. Bei diesem Leitungsmittel kann es sich insbesondere um eine Kapillare handeln. Der Binder, insbesondere der Monomer, fließt aus dem Vorratsbehälter durch das Ventilmittel und das Leitungsmittel in die Vorrichtung, insbesondere in den Mischzylinder. Dort findet eine Vermischung des Binders, insbesondere des Monomers, mit dem Knochenzementpulver statt, um den Knochenzement zu bilden.

Weitere Einzelheiten und Vorteile der Erfindung ergeben sich aus den Ansprüchen, der nachfolgenden Beschreibung und den Zeichnungen. In den Zeichnungen ist die Erfindung in einem Ausführungsbeispiel dargestellt. Es zeigen:
- Fig. 1: eine schematische Schnittzeichnung eines erfindungsgemäßen Knochenzementsystems,
- Fig. 2: ein erfindungsgemäßes Kolbensystem in einer Sterilisationsstellung und
- Fig. 3: das erfindungsgemäße Kolbensystem in einer Mischstellung.

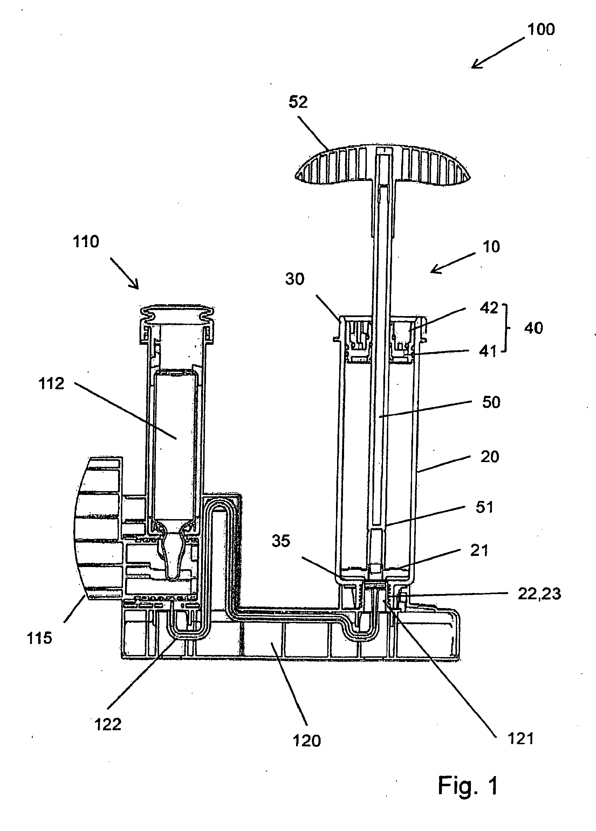

In Figur 1 ist ein erfindungsgemäßes Knochenzementsystem 100 dargestellt. Das Knochenzementsystem 100 weist eine Vorrichtung zum Mischen und Austragen von Knochenzement auf. Diese Vorrichtung 10 ist in dem dargestellten Ausführungsbeispiel gelagert auf einem Basiselement 120. Jenes Basiselement 120 trägt auch ein Vorratselement 110 für einen Binder, insbesondere einen Monomer. Das Knochenzementsystem 100 dient zum Mischen des Knochenzements. Dazu wird Knochenzementpulver in einen Mischzylinder 20 der Vorrichtung 10 eingefüllt. Jenes Knochenzementpulver kann dann mit dem Binder, insbesondere einem Monomer, vermischt werden, um Knochenzement zu bilden. Im Folgenden soll nur auf die Nutzung von Monomer verwiesen werden, was allerdings nicht als eine Einschränkung verstanden werden soll. Bei bekannten Knochenzementsystemen hat es sich als nachteilig erwiesen, dass die desinfizierten Knochenzementsysteme kurz vor dem Zusammenführen des Monomers und des Knochenzementpulvers noch einmal mit der Umgebung in Kontakt treten und dadurch Verunreinigungen, insbesondere Keime, in das Knochenzementpulver eintreten können. Dieser Nachteil wird durch die erfindungsgemäße Vorrichtung 10 sowie das hier offenbarte Knochenzementsystem 100 überwunden.

Die erfindungsgemäße Vorrichtung 10 zum Mischen und Austragen von Knochenzement weist einen Mischzylinder 20 auf. In dem Mischzylinder 20 ist eine Betätigungsstange 50 angeordnet, welche über einen Handgriff 52 axial bewegt werden kann. Wie dargestellt, weist die Betätigungsstange 50 an einem Ende einen Mischkolben 21 auf, der für eine Vermengung des Knochenzementpulvers und des Monomers genutzt werden kann. An einem ersten Zylinderende 30 ist ein Kolbensystem 40 angeordnet. Durch das Kolbensystem 40 wird die Betätigungsstange 50 gedichtet herausgeführt. Die Besonderheit der erfindungsgemäßen Vorrichtung 10 besteht darin, dass das Kolbensystem 40 axial beweglich ist und zweiteilig aufgebaut ist. Das Kolbensystem 40 weist einen Sterilisationskolben 41 und einen Dichtungskolben 42 auf.

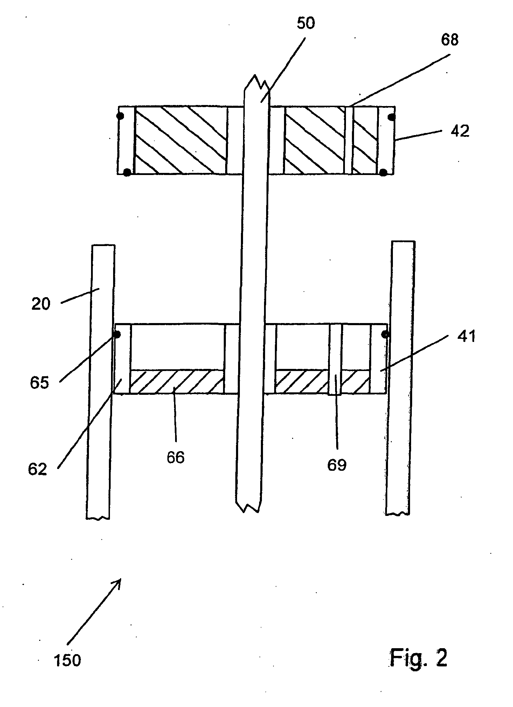

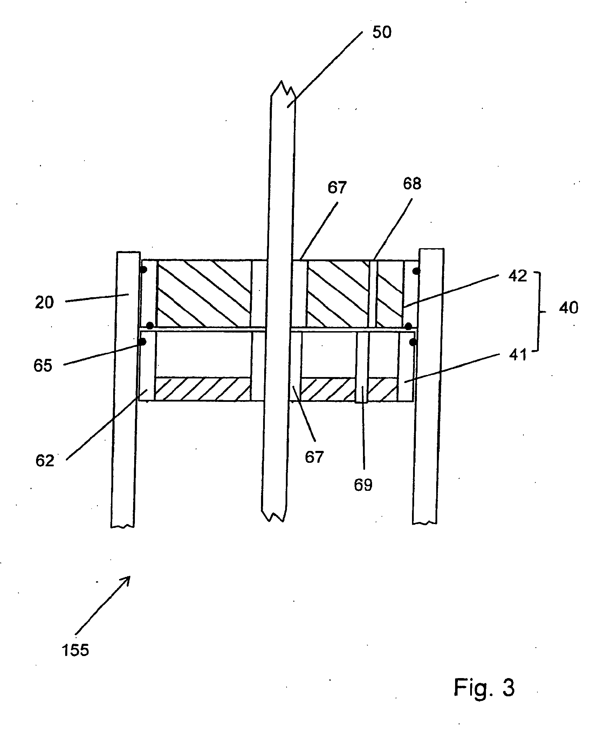

Die Figuren 2 und 3 sollen die Nutzung des zweiteiligen Kolbensystems 40 verdeutlichen. Vor der Auslieferung des Knochenzementsystems 100 wird der Mischzylinder 20 der Vorrichtung 10 vom Hersteller mit Knochenzementpulver befüllt. Im Anschluss wird der gasdurchlässige Sterilisationskolben 41 in den Mischzylinder 20 eingesetzt. Die sich so ergebende Sterilisationsstellung 150 ist in Figur 2 dargestellt. Der Dichtungskolben 42 ist in der Sterilisationsstellung 150 nicht in den Mischzylinder 20 eingeführt, sondern vielmehr auf der Betätigungsstange 50 in Richtung des Handgriffs 52 verschoben. In der Sterilisationsstellung 150 kann der Hersteller den Knochenzement, die Vorrichtung 10 und das Knochenzementsystem 100 mit einem Fluid sterilisieren. Vorzugsweise wird für die Sterilisation Ethylenoxid verwendet. Das Ethylenoxid kann durch den gasdurchlässigen Sterilisationskolben in den Mischzylinder einströmen und das Knochenzementpulver durchspülen. Der Sterilisationskolben 41 weist eine siebartige Struktur 66 auf. Diese siebartige Struktur 66 ermöglicht ein Einströmen des Sterilisationsgases in den Mischzylinder 20. Vorteilhafterweise ermöglicht die siebartige Struktur 66 nur ein Einströmen von Partikeln, die kleiner als 5 µm sind. Dadurch wird verhindert, dass Verunreinigungen in das Knochenzementpulver einströmen können. Nach der Sterilisation des Knochenzementsystems und/oder der Vorrichtung wird diese im Allgemeinen verpackt und an den medizinischen Anwender versendet. Im Rahmen einer Operation kann der medizinische Anwender dann das Knochenzementsystem 100 und/oder die Vorrichtung 10 der Verpackung entnehmen. Zum Mischen des Knochenzementpulvers mit dem Monomer wird die Vorrichtung 10 aus der Sterilisationsstellung 150 in eine Mischstellung 155 überführt, die in Figur 3 dargestellt ist. Im Rahmen der Mischstellung 155 wird der zweite Teil des Kolbensystems 40 - der Dichtungskolben 42 - axial in Richtung des Mischzylinders 40 auf der Betätigungsstange 50 verschoben. Der Dichtungskolben 42 kann auf den Sterilisationskolben aufgesteckt werden. Dazu können beide Kolben 41,42 entsprechende Mittel aufweisen, um eine kraft- und/oder formschlüssige Verbindung zu erzielen. Im Anschluss an die Überführung des Kolbensystems 40 in die Mischstellung 155 wird die Vorrichtung 10 an ein Vakuumsystem angeschlossen. Dazu weist das Kolbensystem 40 einen Vakuumanschluss 68 auf. Dieser Vakuumanschluss 68 ist in dem Dichtungskolben 42 angeordnet. Da der Sterilisationskolben 41 gasdurchlässig ist, bedarf dieser keines Vakuumanschlusses. Der Vakuumanschluss 68 wird genutzt, um im Inneren des Mischzylinders 20 einen Unterdruck zu erzielen.

Wie die Figur 1 verdeutlicht, ist Teil des Knochenzementsystems auch das Vorratselement 110. Das Vorratselement 110 lagert einen Vorratsbehälter 112 für den Monomer. Über ein Ventilmittel 115 kann ein Ausfluss des Monomers aus dem Vorratsbehälter 112 gesteuert und/oder ausgelöst werden. Vorteilhafterweise handelt es sich bei dem Vorratsbehälter 112 um einen Glasbehälter, der im Kopfbereich durch das Ventilmittel 115 geöffnet wird. Der Monomer fließt dann durch ein Leitungsmittel 122 von dem Vorratsbehälter 112 in den Mischzylinder 20. Das Überströmen des Monomers wird dadurch verstärkt, dass im Mischzylinder 20 ein Unterdruck herrscht. Mittels der Betätigungsstange und des Mischkolbens 21 ist dann ein leichtes und einfaches Vermischen des Knochenzementpulvers und des Monomers möglich. Nach fertiger Mischung kann die Vorrichtung 10 von dem Basiselement 120 abgeschraubt werden. Dazu weist das Basiselement 120 ein Koppelmittel 121 auf, welches mit einem Anschlussmittel 22 des Mischkolbens zusammenwirkt. Nach Trennung der Vorrichtung 10 von dem Basiselement 120 wird die Betätigungsstange 50 axial derart verschoben, dass der Mischkolben 21 am Kolbensystem 40 zum Liegen kommt. Anschließend lässt sich die Betätigungsstange an der Sollbruchstelle 51 abknicken. Die Vorrichtung 10 kann nunmehr in eine Zementierpistole integriert werden. Durch Betätigung der Zementierpistole wird eine Zahnstange mit Teller in Richtung des Kolbensystems 40 bewegt. Vorteilhafterweise ist das Kolbensystem 40 nicht nur für die Sterilisation und Mischung des Knochenzements zu nutzen, sondern kann auch zum Austragen des Knochenzements genutzt werden. Dazu ist das Kolbensystem 40 axial beweglich ausgestaltet. Dazu ist das Kolbensystem 40 axial in den Mischzylinder 20 einrückbar. So wird erreicht, dass der aus Knochenzementpulver und dem Monomer gemischte Knochenzement durch eine Austragsöffnung 23 ausgetragen werden kann.

Sowohl der Sterilisationskolben 41 als auch der Dichtungskolben 42 des erfindungsgemäßen Kolbensystems 40 weisen Abdichtungsmittel 65 auf. Diese verhindern, dass Umgebungseinflüsse in das Innere des Mischzylinders einströmen können. Darüberhinaus weisen die beiden Kolben 41,42 des Kolbensystems 40 Führungsmittel 67 auf, die zur Abstützung gegenüber der Betätigungsstange 50 dienen. So wird ein Verkanten der beiden Kolben 41,42 verhindert. Damit beim Befüllen der Vorrichtung 10 mit dem Knochenzementpulver der Sterilisationskolben 41 nicht aus dem Mischzylinder 20 entnommen werden muss, kann der Sterilisationskolben 41 über eine Befüllöffnung 69 verfügen. Durch diese insbesondere reversibel verschließbare Befüllöffnung kann das Knochenzementpulver in das Innere des Mischzylinders 20 eingefüllt werden.

### Bezugszeichen

- 10: Vorrichtung

- 20: Mischzylinder
- 21: Mischkolben
- 22: Anschlussmittel
- 23: Austragsöffnung

- 30: erstes Zylinderende
- 35: zweites Zylinderende

- 40: Kolbensystem
- 41: Sterilisationskolben
- 42: Dichtungskolben

- 50: Betätigungsstange
- 51: Sollbruchstelle
- 52: Handgriff

- 62: Führungsmittel
- 65: Abdichtungsmittel
- 66: siebartige Struktur
- 67: Führungsmittel
- 68: Vakuumanschluss
- 69: Befüllöffnung

- 100: Knochenzementsystem

- 110: Vorratselement
- 112: Vorratsbehälter
- 115: Ventilmittel

- 120: Basiselement
- 121: Koppelmittel
- 122: Leitungsmittel

- 150: Sterilisationsstellung
- 155: Mischstellung

## Patentansprüche

1. Vorrichtung (10) zum Mischen und Austragen von Knochenzement,
mit einem Mischender (20), in welchem ein Mischkolben (21) angeordnet ist, wobei
der Mischkolben (21) mittels einer an einem ersten Zylinderende (30) gedichtet herausgeführten Betätigungsstange (50) axial bewegbar ist,
und mit einem im Bereich des ersten Zylinderendes (30) angeordneten und auf der Betätigungsstange (50) axial bewegbaren Dichtungskolben (42), der den Mischzylinder (20) gasdicht abschließt, wobei
im Bereich des ersten Zylinderendes (30) zwischen dem Mischkolben (21) und dem Dichtungskolben (42) ein Sterilisationskoiben (41) angeordnet ist,
der getrennt von dem Dichtungskolben (42) auf der Betätigungsstange (50) axial bewegbar ist, und den Mischzylinder (20) gasdurchlässig abschließt, wobei
der Sterilisationskolben (41) mit dem Dichtungskolben (42) ein zweiteiliges Kolbensystem (40) bildet,
wobei in einer Mischstellung (155) der Dichtungskolben (42) den Mischzylinder (20) gasdicht abschließt, um ein Durchspülen des Knochenzementpulvers mit einem Binder, insbesondere einem Monomer, zu ermöglichen
**dadurch gekennzeichnet, dass**
der Sterilisationskolben (41) ein gasdurchlässiges Gitter aufweist, das Partikel, die kleiner als 5 µm sind, durchlässt, und
in einer Sterilisationsstellung (150) der Sterilisationskolben (41) den Mischzylinder (20) gasdurchlässig abschließt, um ein Durchspülen eines Knochenzementpulvers mit einem Sterilisationsmittel zu ermöglichen.

2. Vorrichtung (10) nach Anspruch 1, **dadurch gekennzeichnet, dass** das Kolbensystem (40) die Betätigungsstange (50) umschließt.

3. Vorrichtung (10) nach einem der vorherigen Anspruche, **dadurch gekennzeichnet, dass** der Dichtungskolben (42) auf den Sterilisationskolben (41) aufsteckbar ist.

4. Vorrichtung (10) nach einem der vorherigen Ansprüche, **dadurch gekennzeichnet, dass** das Kolbensystem (40) axial in den Mischzylinder (20) eindrückbar ist, um einen aus dem Knochenzementpulver und dem Binder, insbesondere einem Monomer, gemischten Knochenzement durch eine Austragsöffnung (23) auszutragen.

5. Vorrichtung (10) nach Anspruch 4, **dadurch gekennzeichnet, dass** die Austragsöffnung ein Anschlussmittel (22), insbesondere ein Anschlussgewiride, aufweist.

6. Vorrichtung (10) nach einem der vorherigen Anspruche, **dadurch gekennzeichnet, dass** die Betätigungsstange (50) eine Sollbruchstelle (51) aufweist.

7. Vorrichtung (10) nach einem der vorherigen Ansprüche, **dadurch gekennzeichnet, dass** der Sterilisationskolben (41) eine verschließbare, insbesondere reversibel verschließbare Befüllöffnung (69) aufweist.

8. Vorrichtung (10) nach einem der vorherigen Anspruche, **dadurch gekennzeichnet, dass** das Kolbensystem (40) und/oder der Sterilisationskolben (41) und/oder der Dichtungskolben (42) wenigstens einen Vakuumanschluss (68) aufweisen.

9. Knochenzementsystem (100) mit einer Vorrichtung (10) zum Mischen und Austragen von Knochenzement nach einem der vorherigen Ansprüche, einem Vorratselement (110) für einen Binder, insbesondere einen Monomer, und einem Basiselement (120), wobei das Basiselement (120) die Vorrichtung (10) und das Vorratselement (110) lagert.

10. Knochenzementsystem (100) nach Anspruch 9, **dadurch gekennzeichnet, dass** das Basiselement (120) ein Koppelmittel (121) aufweist für eine kraft- und/oder formschlüssige Verbindung mit der Vorrichtung (10), insbesondere einer Austragsöffnung (23) der Vorrichtung (10).

11. Knochenzementsystem (100) nach einem der vorherigen Ansprüche 9 oder 10, **dadurch gekennzeichnet, dass** das Vorratselement (110) einen Vorratsbehälter (112) für den Binder, insbesondere den Monomer, lagert, insbesondere, dass der Vorratsbehälter (112) ein Glasbehälter ist.

12. Knochenzementsystem (100) nach einem der vorherigen Ansprüche 9 bis 11, **dadurch gekennzeichnet, dass** das Vorratselement (110) ein Ventlimittel (115) aufweist, um einen Ausfluss des Monomers aus dem Vorratsbehälter (112) zu steuern und/oder auszulösen.

13. Knochenzementsystem (100) nach einem der vorherigen Ansprüche 9 bis 12, **dadurch gekennzeichnet, dass** das Basiselement ein Leitungsmittel (122) aufweist, wobei durch das Leitungsmittel (122) der Monomer aus dem Vorratsbehälter (112) in die Vorrichtung (10), insbesondere den Mischzylinder (20), strömt.

## Claims

1. Device (10) for mixing and dispensing bone cement,
comprising a mixing cylinder (20), in which a mixing plunger (21) is arranged, whereby
the mixing plunger (21) is mobile in axial direction by means of an actuation rod (50) that is guided out in tightly sealed manner at a first cylinder end (30),
and comprising a sealing plunger (42) that is arranged in the region of the first cylinder end (30) and is mobile in axial direction on the actuation rod (50) and seals the mixing cylinder (20) in gas-tight manner, whereby
a sterilisation plunger (41) is arranged in the region of the first cylinder end (30) between the mixing plunger (21) and the sealing plunger (42) and is
mobile in axial direction on the actuation rod (50) separate from the sealing plunger (42) and seals the mixing cylinder (20) in gas-permeable manner, whereby
the sterilisation plunger (41) and the sealing plunger (42) jointly form a two-part plunger system (40),
whereby, in a mixing position (155), the sealing plunger (42) seals the mixing cylinder (20) in gas-tight manner in order to enable a bone cement powder to be rinsed [AK1] by a binding agent, in particular by a monomer,
**characterised in that**
the sterilisation plunger (41) comprises a gas-permeable grid that allows particles smaller than 5 µm to pass, and,
in a sterilisation position (150), the sterilisation plunger (41) seals the mixing cylinder (20) in a gas-permeable manner in order to enable a bone cement powder to be rinsed [AK2] by a sterilisation agent.

2. Device (10) according to claim 1, **characterised in that** the plunger system (40) surrounds the actuation rod (50).

3. Device (10) according to any one of the preceding claims, **characterised in that** the sealing plunger (42) can be plugged onto the sterilisation plunger (41).

4. Device (10) according to any one of the preceding claims, **characterised in that** the plunger system (40) can be pushed into the mixing cylinder (20) in axial direction to dispense, through a dispensing opening (23), a bone cement produced by mixing the bone cement powder and the binding agent, in particular a monomer.

5. Device (10) according to claim 4, **characterised in that** the dispensing opening comprises a connecting means (22), in particular a connection thread.

6. Device (10) according to any one of the preceding claims, **characterised in that** the actuation rod (50) comprises a pre-determined breakage site (51).

7. Device (10) according to any one of the preceding claims, **characterised in that** the sterilisation plunger (41) comprises a closable, in particular a reversibly closable, filling opening (69).

8. Device (10) according to any one of the preceding claims, **characterised in that** the plunger system (40) and/or the sterilisation plunger (41) and/or the sealing plunger (42) comprise(s) a vacuum connector (68).

9. Bone cement system (100) comprising a device (10) for mixing and dispensing bone cement according to any one of the preceding claims, a reservoir element (110) for a binding agent, in particular a monomer, and a base element (120), whereby the base element (120) supports, like a bearing, [AK3] the device (10) and the reservoir element (110).

10. Bone cement system (100) according to claim 9, **characterised in that** the base element (120) comprises a coupling means (121) for a non-positive fit-like and/or positive fit-like connection to the device (10), in particular to a dispensing opening (23) of the device (10).

11. Bone cement system (100) according to any one of the preceding claims 9 or 10, **characterised in that** the reservoir element (110) supports, like a bearing, [AK4] a reservoir container (112) for the binding agent, in particular the monomer, in particular in that the reservoir container (112) is a glass container.

12. Bone cement system (100) according to any one of the preceding claims 9 to 11, **characterised in that** the reservoir element (110) comprises a valve means (115) for controlling and/or triggering an outflow of the monomer from the reservoir container (112).

13. Bone cement system (100) according to any one of the preceding claims 9 to 12, **characterised in that** the base element comprising a conduit means (122), whereby the monomer flows from the reservoir container (112) through the conduit means (122) into the device (10), in particular into the mixing cylinder (20).

## Revendications

1. Dispositif (10) de mélange et de distribution de ciment osseux,
comprenant un cylindre de mélange (20) dans lequel un piston de mélange (21) est disposé, dans lequel
le piston de mélange (21) est mobile axialement au moyen d'une tige d'actionnement sortie (50) serrée à une première extrémité de cylindre (30),
et comprenant un piston d'étanchéité (42) disposé dans la région de la première extrémité de cylindre (30) et mobile axialement sur la tige d'actionnement (50) qui ferme le cylindre de mélange (20) de manière étanche au gaz, dans lequel
un piston de stérilisation (41) est disposé dans la région de la première extrémité de cylindre (30) entre le piston de mélange (21) et le piston d'étanchéité (42),
lequel est mobile axialement séparément du piston d'étanchéité (42) sur la tige d'actionnement (50) et ferme le cylindre de mélange (20) de manière perméable au gaz, dans lequel
le piston de stérilisation (41) forme avec le piston d'étanchéité (42) un système de pistons en deux parties (40),
dans lequel le piston d'étanchéité (42) ferme le cylindre de mélange (20) de manière étanche au gaz dans une position de mélange (155) pour permettre un rinçage de la poudre de ciment osseux avec un liant, notamment un monomère,
**caractérisé en ce que**
le piston de stérilisation (41) présente une grille perméable au gaz qui laisse passer des particules qui sont inférieures à 5 µm, et
le piston de stérilisation (41) ferme le cylindre de mélange (20) de manière perméable au gaz dans une position de stérilisation (150) pour permettre un rinçage d'une poudre de ciment osseux avec un agent de stérilisation.

2. Dispositif (10) selon la revendication 1, **caractérisé en ce que** le système de pistons (40) entoure la tige d'actionnement (50).

3. Dispositif (10) selon l'une quelconque des revendications précédentes, **caractérisé en ce que** le piston d'étanchéité (42) peut être enfiché sur le piston de stérilisation (41).

4. Dispositif (10) selon l'une quelconque des revendications précédentes, **caractérisé en ce que** le système de pistons (40) peut être enfoncé axialement dans le cylindre de mélange (20) pour distribuer un ciment osseux mélangé à partir de la poudre de ciment osseux et du liant, notamment d'un monomère à travers une ouverture de distribution (23).

5. Dispositif (10) selon la revendication 4, **caractérisé en ce que** l'ouverture de distribution présente un moyen de raccord (22), notamment un filetage de raccord.

6. Dispositif (10) selon l'une quelconque des revendications précédentes, **caractérisé en ce que** la tige d'actionnement (50) présente un point destiné à la rupture (51).

7. Dispositif (10) selon l'une quelconque des revendications précédentes, **caractérisé en ce que** le piston de stérilisation (41) présente une ouverture de remplissage verrouillable (69), notamment verrouillable de manière réversible.

8. Dispositif (10) selon l'une quelconque des revendications précédentes, **caractérisé en ce que** le système de pistons (40) et/ou le piston de stérilisation (41) et/ou le piston d'étanchéité (42) présentent au moins un raccord sous vide (68).

9. Système de ciment osseux (100) avec un dispositif (10) de mélange et de distribution de ciment osseux selon l'une quelconque des revendications précédentes, un élément de réserve (110) pour un liant, notamment un monomère, et un élément de base (120), dans lequel l'élément de base (120) reçoit le dispositif (10) et l'élément de réserve (110).

10. Système de ciment osseux (100) selon la revendication 9, **caractérisé en ce que** l'élément de base (120) présente un moyen d'accouplement (121) pour une connexion par adhérence et/ou conjugaison de formes au dispositif (10), notamment à une ouverture de distribution (23) du dispositif (10).

11. Système de ciment osseux (100) selon l'une quelconque des revendications précédentes 9 ou 10, **caractérisé en ce que** l'élément de réserve (110) reçoit un récipient de réserve (112) pour le liant, notamment le monomère, notamment que le récipient de réserve (112) est un récipient en verre.

12. Système de ciment osseux (100) selon l'une quelconque des revendications précédentes 9 à 11, **caractérisé en ce que** l'élément de réserve (110) présente un moyen de soupape (115) pour commander et/ou déclencher une décharge du monomère du récipient de réserve (112).

13. Système de ciment osseux (100) selon l'une quelconque des revendications précédentes 9 à 12, **caractérisé en ce que** l'élément de base présente un moyen de conduite (122), dans lequel le monomère s'écoule du récipient de réserve (112) dans le dispositif (10), notamment le cylindre de mélange (20), à travers le moyen de conduite (122).
